# EUROPEAN PATENT SPECIFICATION

(11) **EP 1 166 764 B1**
(45) Date of publication and mention of the grant of the patent: **10.12.2003**
(21) Application number: 00307069.5
(22) Date of filing: 17.08.2000
(51) Int. Cl.: A61K 7/48

(54) **Method of promoting nutrient, water and/or oxygen uptake into the skin**
Verfahren zur Förderung der Aufnahme von Nährstoffen, Wasser und/oder Sauerstoff in die Haut
Procédé favorisant l'absorption de substances nutritives, d'eau et/ou d'oxygène dans la peau

(30) Priority: 29.06.2000 US 606490
(43) Date of publication of application: 02.01.2002
(73) Proprietor: JOHNSON & JOHNSON CONSUMER COMPANIES, INC., Skillman, NJ 08558 (US)
(72) Inventor: Shapiro, Stanley S., Livingston, NJ 07039 (US); Martin, Katharine M., Ringoes, NJ 08551 (US); Saliou, Claud, Princeton, NJ 08542 (US); Wiegand, Benjamin C., Newtown, PA 18940 (US); Packer, Lester, Orinda, CA 94563 (US)
(74) Representative: Fisher, Adrian John

(56) References cited:
- EP-A- 0 974 342
- FR-A- 1 248 192
- FR-A- 2 738 742

## Description

### FIELD OF THE INVENTION

The present invention relates to the cosmetic use of a mineral water for promoting the uptake of nutrients and/or oxygen into the skin.

### BACKGROUND OF THE INVENTION

Skin cells uptake fluids and nutrients from the extracellular environment through the process of pinocytosis. During pinocytosis, cells ingest such fluids and nutrient solutes via small vesicles (e.g., generally less than 150 nm in diameter) made up of the cell's plasma membrane. The cell's plasma membrane encapsulates the extracellular fluid containing the nutrients. The resulting vesicle then detaches from the cell membrane and travels into the intracellular region of the cell. Pinocytosis is generally known as a "constitutive" process because it is continuous in that the cell is always ingesting pieces of its plasma membrane along with extracellular fluids and nutrients. The purpose of many skin care compositions (e.g., moisturizing lotions containing emollients and humectants) is to hydrate or induce nutrient uptake into the skin (e.g., nourish the skin).

FR-A-1 248 192 discloses a method for hydrating and/or nourishing the skin comprising applying a composition comprising mineral water Evian. FR-A-2 738 742 discloses the use of mineral water Vichy for the preparation of a pharmaceutical composition for treating disorders associated with an excess of synthesis and/or liberation of substance P.

The skin serves as a barrier between the outside environment and the inside body. Maintaining an effective barrier in any circumstances requires a constant renewal of the skin's outer layer, the production of a tight but yet elastic extracellular matrix, and the participation of endogenous natural defences. Nonetheless, these processes are energy-dependent and require oxygen. Because of its unique location, the skin can uptake oxygen from two different sources namely the bloodstream and the air. While the dermis receives its oxygen from the bloodstream carrying oxyhemoglobin-loaded erythrocytes, the epidermis uptakes it directly from the atmosphere. In fact, it is well established that the uptake of oxygen by the skin is equivalent to its carbon dioxide excretion.

The present invention relates to the cosmetic use of a mineral water for nutrient uptake and/or oxygen uptake in the skin (e.g., in skin care compositions).

### SUMMARY OF THE INVENTION

The invention features the cosmetic use of a mineral water for promoting the uptake of nutrients and/or oxygen into the skin (e.g., into skin cells) wherein said mineral water has a mineralization of at least 200 mg/L (e.g., from 300 mg/L to about 1000 mg/L), wherein said mineral water comprises at least about 10 mg/L of calcium and at least about 5 mg/L of magnesium.

The invention further provides a composition comprising (i) mineral water having a mineralization of at least about 200 mg/L, wherein said mineral water comprises at least about 10 mg/L of calcium and at least about 5 mg/L magnesium; and (ii) a compound selected from creatine, carnitine, pyruvic acid and cosmetically acceptable salts or esters thereof, for use as a cosmetic for promoting the uptake of a nutrient, and/or oxygen into the skin.

What is meant by promoting the uptake is either (i) increasing the amount of water, nutrient, and/or oxygen stored in the skin or (ii) increasing the rate by which water, nutrient, and/or oxygen is taken in by the skin. In one embodiment, the increase in amount or rate is at least about 5%, such as at least about 20%.

In one embodiment, the composition further comprises a humectant or an emollient.

In a further embodiment, the invention provides the use of a mineral water having a mineralization of at least about 200 mg/L, wherein said mineral water comprises at least about 10 mg/L of calcium and at least about 5 mg/L of magnesium in the manufacture of a medicament for therapeutic treatment of the skin by promoting the uptake of a nutrient, and/or oxygen into the skin.

The promotion of water or nutrient uptake provides healthier skin, e.g., enhances skin firmness and elasticity and enhances skin barrier function. The promotion of oxygen uptake makes the skin more radiant, enhances skin glow, and evens skin tone.

Other features and advantages of the present invention will be apparent from the detailed description of the invention and from the claims.

### DETAILED DESCRIPTION OF THE INVENTION

It is believed that one skilled in the art can, based upon the description herein, utilize the present invention to its fullest extent. The following specific embodiments are to be construed as merely illustrative, and not limitative of the remainder of the disclosure in any way whatsoever.

Unless defined otherwise, all technical and scientific terms used herein have the same meaning as commonly understood by one of ordinary skill in the art to which the invention belongs.

The present invention relates to the cosmetic use of a mineral water (e.g., a cosmetic composition comprising mineral water) as disclosed in claims 1 to 4.

The mineral water may be a naturally mineralized water, e.g., a mineral water suitable for consumption, or a thermal spring water, which is often not consumable. Examples of mineral water include, but are not limited to, eau d'Evian (Evian Eau Minerale Naturelle or Evian® Natural Spring Water referred herein as Evian® Mineral Water), eau Volvic, and eaux de Vittel (e.g., Grande Spring or Hepar Spring). Examples of thermal spring waters include eau de la Bourboule, eau d'Enghien-les-bains, eau d'Allevard-les-bains, eau de Digne, eau des Maizieres, eau de Nyrac-les-bains, eau de Lons le Saunier, Eaux Bonnes, eau de Rochefort, eau de Saint Christau, eau des Fumades, eau de Tereau de Vittel, eaux du bassin de Vichy, eau d'Uriage, eau d'Avene, and eau de la Roche Posay.

In one embodiment, the mineral water comprises (a) from 30 mg/L to 150 mg/L of calcium; (b) from 10 mg/L to 50 mg/L of magnesium; (c) from 150 mg/L to 700 mg/L of bicarbonates; (d) from 0.1 mg/L to 5 mg/L of potassium. In a further embodiment, the mineral water additionally comprises (e) from 1 to 20 mg/L of sulfates; (f) from 1 to 10 mg/L of sodium; (g) from 1 mg/L to 10 mg/L of chlorides; and (h) from 1 mg/L to 10 mg/L of nitrates.

In one embodiment, the mineral water is Evian® Mineral Water that comprises: (a) about 78 mg/L of calcium; (b) about 24 mg/L of magnesium, (c) about 357 mg/L of bicarbonates; (d) about 1 mg/L of potassium; (e) about 10 mg/L of sulfates; (f) about 5 mg/L of sodium; (g) about 4 mg/L of chlorides, and (h) from about 1 to about 4 mg/L nitrates.

What is meant by a promoting amount of mineral water is an amount capable of promoting the desired effect (e.g., promoting the uptake of the nutrient, water, or/and oxygen in skin cells). In one embodiment, the composition comprises at least about 0.1%, by weight, of mineral water, e.g., about 10% to about 99%, by weight, of mineral water.

The cosmetic use of the present invention may require the topical administration (e.g. in a composition) of one or more of the following compounds: creatine, carnitine, or pyruvic acid, or a cosmetically acceptable salt or ester thereof. What is meant by cosmetically acceptable salt or ester is one that does not eliminate the therapeutic benefit of the compound (e.g., its hydrating or nourishing properties). Examples of cosmetically acceptable salts, include, but are not limited to, those with cosmetically acceptable organic acids (e.g., acetic, lactic, maleic, citric, malic, ascorbic, succinic, benzoic, methesulfonic, toluenesulfonic, or pamoic acid), as well as polymeric acids (e.g., tannic or carboxymethyl cellulose) and salts with inorganic acids such as a hydrohalic acid (e.g., hydrochloric acid, sulfuric acid, or phosphoric acid). Examples of cosmetically acceptable esters include, but are not limited to, C2-C6 alkyl esters such as methyl esters and ethyl esters. Examples of such compounds include, but are not limited to, creatine monohydrate, creatine hemisulfate, D-carnitine, L-carnitine, L-carnitine hydrochloride, sodium pyruvate, and pyruvic acid methyl ester. As used herein, if the stereochemistry of the compound is not indicated, then the compound includes all stereoisomers, if any.

What is meant by a nutrient is an organic substance occurring in foods that is not synthesized by the body and is necessary in trace amounts for the normal metabolic functioning of the body, such as vitamins, essential amino acids, and essential fatty acids.

Examples of such vitamins include, but are not limited to, vitamin A, a vitamin B (e.g., vitamin B1, vitamin B2, vitamin B6, or vitamin B12), vitamin C, and a vitamin E (e.g., a tocopherol or a tocotrienol), and cosmetically acceptable salts and esters thereof, such a retinyl palmitate, retinyl acetate, tocopherol succinate, and tocopherol acetate.

Examples of such essential amino acids include, but are not limited to, arginine, histidine, isoleucine, leucine, lysine, methionine, phenylalanine, threonine, tryptophan, and valine.

Examples of essential fatty acids include, but are not limited to, linoleate and linolenate.

What is meant by an emollient is a compound that helps to maintain the soft, smooth, and pliable appearance of the skin (e.g., by remaining on the skin surface or in the stratum corneum to act as a lubricant). Examples of emollients can be found on pages 1657-1661 of the International Cosmetic Ingredient Dictionary and Handbook, eds. Wenninger and McEwen, pp. 1612-13, 1626, and 1654-55 (The Cosmetic, Toiletry, and Fragrance Assoc., Washington, D.C., 7^{th} Edition, 1997) (hereinafter "ICI Handbook"), and include, but are not limited to, hexyldecyl stearate and plant, nut, and vegetable oils such as macadamia nut oil, rice bran oil, grape seed oil, palm oil, primrose oil, hydrogenated peanut oil, olive oil and avocado oil.

What is meant by a humectant is a compound intended to increase the water content of the top layers of skin (e.g., hygroscopic compounds). Examples of humectants can be found on pages 1661-1662 of the ICI Handbook and include, but are not limited to, glycerin or trehalose (e.g., α,α- trehalose, β,β-trehalose, α,β-trehalose) or a salt or ester thereof (e.g., trehalose 6-phosphate).

The amount of carnitine or a cosmetically acceptable salt or ester thereof, creatine or a cosmetically acceptable salt or ester thereof, pyruvic acid or a cosmetically acceptable salt or ester thereof, nutrient, emollient, or humectant in the composition varies (e.g., depending on the intended use or the form of the composition) being administered and will typically be present in the composition in an amount from 0.001% to 20%, by weight, of the topically applied composition, e.g., from 0.01% to 10%, by weight, such as from 0.01% to 5%, by weight, of such emollient or humectant and from 0.001% to 10%, by weight, of the topically applied composition, e.g., from 0.01% to 5%, by weight, such as from 0.01% to 1%, by weight, of such carnitine, creatine, pyruvic acid or cosmetically acceptable salt or ester thereof.

Another cosmetically active agent may be administered (e.g. in a composition). What is meant by a "cosmetically active agent" is a compound that has a cosmetic or therapeutic effect on the skin, e.g., agents to treat wrinkles, acne, or to lighten the skin. In one embodiment, the agent is selected from, but not limited to, the group consisting of hydroxy acids, benzoyl peroxide, sulfur resorcinol, ascorbic acid, D-panthenol, hydroquinone, sunscreen agents, anti-inflammatory agents, skin lightening agents, antimicrobial and antifungal agents, estrogens, 2-dimethylaminoethanol, lipoic acid, amino acids such a proline and tyrosine, lactobionic acid, acetyl-coenzyme A, niacin, riboflavin, thiamin, ribose, electron transporters such as NADH and FADH2, botanical extracts such as aloe vera and soy, and derivatives and mixtures thereof. The cosmetically active agent will typically be present in an amount of from 0.001% to 20% by weight of the composition, e.g., 0.01% to 10% such as 0.1% to 5%.

Examples of hydroxy acids include, but are not limited, to (i) alpha-hydroxy acids such as glycolic acid, lactic acid, malic acid, citric acid, and tartaric acid, (ii) beta-hydroxy acids such as salicylic acid, and/or (iii) polyhydroxy acids. See, e.g., European Patent Application No. 273,202.

Examples of derivatives of ascorbic acid include, but are not limited to, ascorbyl palmitate, magnesium ascorbyl phosphate, sodium ascorbyl phosphate, zinc ascorbyl phosphate, ascorbyl glucoside, sodium ascorbate, and ascorbyl polypeptide. An example of a derivative of hydroquinone includes, but is not limited to, arbutin.

The cosmetic use of the present invention can be practiced by topically administering to a mammal, e.g., by the direct laying on or spreading on the skin of a human, the mineral water and any other ingredients in a composition. The compositions (e.g., cosmetic compositions) useful in the subject invention involve formulations suitable for topical application to mammalian skin, a formulation comprising (i) mineral water, (ii) optionally, a safe and effective amount of creatine, carnitine, or pyruvic acid, or a cosmetically acceptable salt or ester thereof, (iii) optionally a nutrient, an emollient, humectant (e.g., trehalose), or other cosmetically active agent(s), and (iv) optionally, a cosmetically-acceptable topical carrier. The term "cosmetically-acceptable topical carrier" refers to a carrier for topical use that is capable of having the mineral water and any other agents dispersed or dissolved therein, and possessing acceptable safety properties. In one embodiment, the cosmetically-acceptable carrier comprises mineral water (e.g., an emulsion where the aqueous phase comprises mineral water or a mineral water based cleanser or spray).

The topical compositions useful in the present invention may be used for a variety of cosmetic uses, including, but not limited to, treating, cleansing, beautifying, or covering the skin or hair of a human. The compositions, thus, may be made into a wide variety of product types. These include, but are not limited to lotions, creams, gels, sticks, sprays, ointments, pastes, mousses, shampoos, cosmetics, and dermal patches. Products include but are not limited to, lip balms, moisturizing and sunscreen lotions/creams, skin cleansing compositions (e.g., facial scrubs), and body mists. These products may comprise several types of carrier systems including, but not limited to single phase solutions (e.g., water, such as mineral water, or oil based solutions), emulsions, and gels.

The topical compositions useful in the present invention formulated as solutions typically include a cosmetically acceptable water, mineral water, and/or organic carriers (e.g. , from 80% to 99.99%, by weight of the composition, such as from 90% to 99%, by weight of the composition, of an acceptable water, mineral water, or organic solvent). Examples of suitable organic solvents include: propylene glycol, polyethylene glycol (200-600), polypropylene glycol (425-2025), glycerol, 1,2,4-butanetriol, sorbitol esters, 1,2,6-hexanetriol, ethanol, isopropanol, butanetriol, sorbitol esters, 1,2,6-hexanetriol, butanediol, and mixtures thereof.

If the topical solution useful in the present invention are formulated as an aerosol and applied to the skin as a spray-on, a propellant is added to a solution composition. Examples of propellants useful herein include, but are not limited to, chlorinated, fluorinated, and chloro-fluorinated lower molecular weight hydrocarbons. Other propellants useful herein can be found in Sagafin, Cosmetics Science and Technology, 2nd Edition, Vol. 2, pp. 443-65 (1972) (hereinafter "Sagafin") and the ICI Handbook pp. 1655.

A lotion can be made from a solution carrier system. Lotions typically comprise from 1% to 20% by weight of the composition (e.g., from 5% to 10%) of an emollient (s) and from 50% to 90% by weight of the composition (e.g., from 60% to 80%) of water, e.g., mineral water.

Another type of product that may be formulated from a solution carrier system is a cream. A cream typically comprises from 5% to 50% by weight of the composition (e.g., from 10% to 20%) of an emollient (s) and from 45% to 85% by weight of the composition (e.g., from 50% to 75%) of water.

Yet another type of product that may be formulated from a solution carrier system is an ointment. An ointment may comprise a simple base of animal or vegetable oils or semi-solid hydrocarbons. Ointments may also comprise absorption ointment bases that absorb water to form emulsions. Ointment carriers may also be water-soluble. An ointment may comprise from about 1% to about 20% by weight of the composition of an emollient(s) plus from 0.1% to 2% by weight of the composition of a thickening agent(s). A more complete disclosure of thickening agents or viscosity increasing agents useful herein can be found in Sagafin pp. 72-73 and the ICI Handbook pp. 1693-97.

If the carrier is formulated as an emulsion (e.g., an oil-in-water, silicone-in-water, water-in-oil, or water-in-silicone emulsion), from 1% to 10% by weight of the composition (e.g., from 2% to 5%) of the carrier system may comprise an emulsifier(s). Emulsifiers may be nonionic, anionic, cationic, or zwitterionic. Suitable emulsifiers are disclosed in, for example, U.S. Patent No. 3,755,560, U.S. Patent No. 4,421,769, McCutcheon's Detergents and Emulsifiers, North American Edition, pp. 317-24 (1986), and the ICI Handbook, pp. 1673-86.

Lotions and creams can also be formulated as emulsions. Typically, such emulsions may comprise from 0.5% to 5% by weight of the composition of an emulsifier(s). Creams may typically comprise from 1% to 20% by weight of the composition (e.g., from 5% to 10%) of an emollient(s); from 20% to 80% by weight of the composition (e.g., from 30% to 70%) of water such as mineral water; and from 1% to 10% by weight of the composition (e.g. , from 2% to 5%) of an emulsifier(s).

Two phase emulsion skin care preparations, such as lotions and creams, of the oil-in-water type and water-in-oil type are well-known in the cosmetic art and are useful in the subject invention. Triphase emulsion compositions, such as the water-in-oil-in-water type, as disclosed in U.S. Patent No. 4,254,105, are also useful in the subject invention. In general, such triphase emulsions contain water, emollients, and emulsifiers as essential ingredients. Triple emulsion carrier systems comprising an oil-in-water-in-silicone fluid emulsion composition, as disclosed in U.S. Patent No. 4,960,764, may also be useful in the subject invention.

The cosmetic use of the present invention may involve administering a composition containing one or more of the following: antioxidants (e.g., ascorbic acid, tocopherols, polyphenols, tocotrienols, BHA, and BHT), chelating agents (e.g., EDTA), and preservatives (e.g., parabens). Examples of suitable antioxidants, preservatives, and chelating agents are listed in pp. 1612-13, 1626, and 1654-55 of the ICI Handbook. In addition, the topical compositions useful herein can contain conventional cosmetic adjuvants, such as dyes, opacifiers (e.g. , titanium dioxide), pigments, and fragrances.

The compositions and cosmetic formulations for use in the methods of the present invention may be prepared using methodology that is well known by an artisan of ordinary skill. The following is a description of the testing and manufacturing of cosmetic compositions of the present invention.

### Example 1: Mineral Water Containing Carnitine, Trehalose, and Sodium Pyruvate

A composition containing Evian® Mineral Water (Evian, France), carnitine, sodium pyruvate, and trehalose was manufactured using the ingredients listed in Table I.

**TABLE I**

| **INGREDIENTS** | **% Weight** |
|---|---|
| L-Carnitine | 1 |
| Sodium Pyruvate | 1 |
| Trehalose | 1 |
| D-Panthenol (75%)/ Water (25%) | 1.3 |
| Magnesium Ascorbyl Phosphate | 1 |
| L-Proline | 1 |
| Mineral Water | 72.7 |
| Pentylene Glycol | 20 |
| Phenoxyethanol | 1 |

The mineral water was first heated to 30°C. The other ingredients were then added and dissolved one by one under mixing conditions. The pentylene glycol was obtained from Dragoco Gerberding & Co. (Holzminden, Germany) under the tradename Hydrolite®-5.

### Example 2- Sunscreen Moisturizer

A sunscreen moisturizing composition containing the composition of Example 1 and the sunscreen octyl methoxycinnamate is manufactured using the ingredients listed in Table II.

**Table II**

| **INGREDIENTS** | **% Weight** |
|---|---|
| **Water Phase Ingredients** | |
| Mineral Water | q.s. 100 |
| Disodium EDTA | 0.1 |
| Glycerin | 3 |
| Butylene glycol | 3 |
| Carbomer | 0.25 |
| Acrylate-C10-30 alkyl acrylate crosspolymer | 0.07 |
| Glyceryl polymethacrylate 67% / water 32% / propylene glycol 1% | 5 |
| Propyl paraben | 0.201 |
| Methyl paraben | 0.35 |
| Phenoxyethanol | 0.584 |

| **Oil Phase Ingredients** | |
|---|---|
| Cetearyl alcohol | 1 |
| C12-C15 alkyl benzoate | 4 |
| Potassium cetyl phosphate | 1.5 |
| PEG-100 stearate 50%/ glyceryl stearate 50% | 0.3 |
| Di-C12-13 alkyl malate | 5 |
| Talc | 1 |
| Phenoxyethanol | 0.365 |
| Propyl paraben | |
| Methyl paraben | |
| Iodopropynyl butylcarbamate 10%/ PEG-4 laurate 90% | 0.1 |
| Octyl methoxycinnamate | 7.5 |
| Butyl methoxydibenzoylmethane | 3 |
| Tocopheryl acetate | 1 |

| **Tromethamine Mixture** | |
|---|---|
| Mineral Water | 2 |
| Tromethamine | 0.3 |

| **Trehalose Mixture** | |
|---|---|
| Mineral Water | 2 |
| Trehalose | 0.25 |

| **Post Addition Ingredients** | |
|---|---|
| Cyclomethicone | 2 |
| Composition of Example 1 | 1 |
| Evening primrose oil | 0.01 |
| Fragrance | 0.3 |

To form the water phase, the mineral water (Evian® Mineral Water, Evian, France) of the Water Phase Ingredients was heated to 85°C and stirred for about 15 minutes in a first beaker. The disodium EDTA, glycerin, and butylene glycol were then added to the first beaker and stirred for an additional 10 minutes. The first beaker was then cooled to 82°C. Next, the carbomer and the acylate-C10-30 alkyl acrylate crosspolymer were dispersed in the mixture in the first beaker and stirred for about 25 minutes until the mixture gelified. The remaining Water Phase Ingredients were then added to the first beaker and mixed.

To form the oil phase, the Oil Phase Ingredients were added to a second beaker, heated to 85°C, and mixed for 15 minutes. The oil phase mixture in the second beaker was then added to the first beaker under mixing conditions to form an emulsion. The emulsion was then cooled to 25°C and neutralized with the Tromethamine Mixture. Next, the cyclomethicone was mixed into the emulsion for 15 minutes. Lastly, the Trehalose Mixture, the composition of Example 1, the evening primrose oil, and the fragrance were added tc che resulting mixture and mixed until uniform.

### Example 3: Promotion of Pinocytosis with Mineral Water

The effect of mineral water was evaluated and quantified in primary human epidermal keratinocytes (adult) to determine its promotion of pinocytosis. Pinocytosis is assessed by the active uptake of Oregon-Green® 488 dye-conjugated dextran, which is water soluble and cell membrane impermeable, by keratinocytes. Because of the size of the dextran (10,000 kDa), pinocytosis is the only mechanism allowing its transfer inside the cells, which is detected by fluorescence.

Normal human epidermal keratinocytes (NHEK), obtained from Cascade Biologics (Portland, OR), were cultured in EpiLife® medium (Cascade Biologics). The NHEK were then seeded in 96-well plates (Costar, Cambridge, MA) for 48-72 hours prior to assaying. The Epilife® medium was replaced 24 hours prior to assay with MCDB 153 medium (Sigma Chemicals Co.; St. Louis, MO) prepared with either Evian® Mineral Water or distilled water from Life Technologies (Rockville, MD). At the same time, the Oregon Green® 488 dye-conjugated dextran (final concentration was 0.5 mg/ml; Molecular Probes, Eugene, OR) was added to the MCDB 153 medium, and the cells were incubated for 24-hours.

At the end of the incubation period, NHEK were washed twice with 200 µl/well phosphate buffered saline (Life Technologies) and incubated with a trypan blue solution (0.25 mg/ml; Sigma Chemicals, Co, St Louis, MO) for 1 min to quench residual external fluorescence. The trypan blue solution was then removed by aspiration, and the cells washed twice with phosphate buffered saline. The fluorescence was subsequently measured using a CytoFluor® Fluorescence Plate Reader (PerSeptive Biosystems, Framingham, MA) set with the following filter-combination: excitation at 485 nm and emission at 530 nm.

Four experiments were run varying the cell seeding concentrations (10,000 cells/cm² - 30,000 cells/cm²) on either clear or black-bottomed 96-well plates. The MCDB 153 medium prepared with mineral water was unexpectedly found to significantly enhance the uptake of the dextran into the cells by 20% (n=129).

### Example 4: Promotion of Water Uptake into Human Skin

A study was performed on 14 human panelists to measure the uptake of water into skin from both distilled water and mineral water (Evian® Mineral Water). Moisturization measurements were made using the Nova™ DPM 9003 (Nova Technology Corporation, Portsmouth, NH). The two products were rotated on the different forearms of the panelists, so that there was an even distribution of products on each forearm during the study. The protocol for the study is recited below.

Five initial conductance measurements were taken before the forearms were soaked. These five values were averaged and recorded. Each volar forearm was soaked in a water solution for five minutes, either with deionized water or with the mineral water. The forearm was then removed from the water bath and was blotted dry. Conductance measurements were then taken to measure hydration levels in the skin. After the conductance measure was taken, adsorption/desorption measurements were taken using the Tagami mechod (Tagami, Journal of Investigative Dermatology, 75:425-28 (1982)). The same was repeated for the other volar forearm.

Mineral water was surprisingly found to enhance skin moisturization by approximately 10-12% as compared to distilled water which is commonly found in other skin care products.

The results of the Tagami method experiments surprisingly demonstrated that mineral water better prepares the skin to accept and hold 15% more moisture than distilled water.

### Example 5: Promotion of Oxygen Uptake into Skin Cells

Seven human panelists were instructed to initially wash both forearms with Purpose® Gentle Cleansing Wash (Johnson & Johnson Consumer Ccmpanies, Inc., Skillman, New Jersey). They were then instructed to mark both forearms with three circles from a template. Prior to further treatment, Diffuse Reflectance Spectrosccpy (DRS) measurements were taken on all six spots. Diffuse Reflectance Spectroscopy (DRS) are made using a DRS Instrument made by Canfield Scientific, Fairfied, NJ. This instrument shines white light onto the skin and measures the intensity of the light that is remitted by the skin. The light is delivered to the skin and transmitted to the detector with a bifurcated fiber bundle. Each measurement is a reflectance spectrum from 400-800 nm and then is analyzed for oxy- and deoxyhemoglobin. The algorithm subtracts first the contribution of epidermal melanin and then fits the spectrum that remains after the subtraction of the melanin contribution with the absorption spectra of oxy and deoxyhemoglobin.

Each volar forearm was then soaked in either distilled water or mineral water (Evian® Mineral Water) for 15 minutes. The right and left volar forearms were randomized among the panelists. After soaking, the arm was patted dry, and allowed to air dry for one minute. Subsequent DRS readings were taken on three spots for each forearm.

The bio-affinity of mineral water was surprisingly found to provide a richer supply of oxygen to the skin, e.g., by increasing the supply of oxyhemoglobin and decreasing deoxyhemoglobin, both by a factor of two compared to distilled water.

It is understood that while the invention has been described in conjunction with the detailed description thereof, that the foregoing description is intended to illustrate and not limit the scope of the invention, which is defined by the scope of the appended claims. Other aspects, advantages, and modifications are within the claims.

## Claims

1. The cosmetic use of a mineral water having a mineralization of at least about 200 mg/L, wherein said mineral water comprises at least about 10 mg/L of calcium and at least about 5 mg/L of magnesium for promoting the uptake of a nutrient and/or oxygen into the skin.

2. The cosmetic use of claim 1, wherein said mineral water comprises (a) from about 30 mg/L to about 150 mg/L of calcium; (b) from about 10 mg/L to about 50 mg/L of magnesium; (c) from about 150 mg/L to about 700 mg/L of bicarbonates; and (d) from about 0.1 mg/L to about 5 mg/L of potassium.

3. The cosmetic use of claim 2, wherein said mineral water further comprises (e) from about 1 to about 20 mg/L of sulphates; (f) from about 1 to about 10 mg/L of sodium; (g) from about 1 mg/L to about 10 mg/L of chlorides; and (h) from about 1 mg/L to about 10 mg/L of nitrates.

4. The cosmetic use of any preceding claim, wherein said mineral water is Evian® Mineral Water.

5. A composition comprising (i) mineral water having a mineralization of at least about 200 mg/L, wherein said mineral water comprises at least about 10 mg/L of calcium and at least about 5 mg/L of magnesium; and (ii) a compound selected from creatine, carnitine, pyruvic acid, and cosmetically acceptable salt or esters thereof for use as a cosmetic for promoting the uptake of a nutrient and/or oxygen into the skin.

6. The composition of claim 5, wherein said mineral water comprises (a) from about 30 mg/L to about 150 mg/L of calcium; (b) from about 10 mg/L to about 50 mg/L of magnesium; (c) from about 150 mg/L to about 700 mg/L of bicarbonates; and (d) from about 0.1 mg/L to about 5 mg/L of potassium.

7. The composition of claim 6, wherein said mineral water further comprises (e) from about 1 to about 20 mg/L of sulphates; (f) from about 1 to about 10 mg/L of sodium; (g) from about 1 mg/L to about 10 mg/L of chlorides; and (h) from about 1 mg/L to about 10 mg/L of nitrates.

8. The composition of one of claims 5-7, wherein said mineral water is Evian® Mineral Water.

9. The use of a mineral water as recited in any of claims 1 to 4 in the manufacture of a medicament for therapeutic treatment of the skin by promoting the uptake of a nutrient or/and oxygen into the skin.

## Patentansprüche

1. Kosmetische Verwendung von Mineralwasser, welches eine Mineralisierung von etwa mindestens 200 mg/l aufweist, worin das besagte Mineralwasser mindestens etwa 10 mg/l Calcium und mindestens etwa 5 mg/l Magnesium zur Förderung der Aufnahme eines Nährstoffs und/oder von Sauerstoff in die Haut umfaßt.

2. Kosmetische Verwendung nach Anspruch 1, worin das besagte Mineralwasser umfaßt (a) von etwa 30 mg/l bis 150 mg/l Calcium; (b) von etwa 10 mg/l bis etwa 50 mg/l Magnesium; (c) von etwa 150 mg/l bis etwa 700 mg/l Bicarbonate; und (d) von etwa 0,1 mg/l bis etwa 5 mg/l Kalium.

3. Kosmetische Verwendung nach Anspruch 2, worin das besagte Mineralwasser weiterhin umfaßt (e) von etwa 1 bis 20 mg/l Sulphate; (f) von etwa 1 bis 10 mg/l Natrium; (g) von etwa 1 mg/l bis etwa 10 mg/l Chlorid; und (h) von etwa 1 mg/l bis etwa 10 mg/l Nitrate.

4. Kosmetische Verwendung nach einem der vorangehenden Ansprüche, worin das besagte Mineralwasser Evian® Mineralwasser ist.

5. Zusammensetzung, umfassend (i) Mineralwasser mit einer Mineralisierung von mindestens etwa 200 mg/l, worin das besagte Mineralwasser mindestens etwa 10 mg/l Calcium und mindestens etwa 5 mg/l Magnesium; und (ii) eine Verbindung ausgewählt aus Kreatin, Karnitin, Brenztraubensäure und kosmetisch akzeptable Salze oder Ester davon umfaßt zur Verwendung als Kosmetikum zur Förderung der Aufnahme eines Nährstoffes und/oder von Sauerstoff in die Haut.

6. Zusammensetzung nach Anspruch 5, worin das besagte Mineralwasser umfaßt (a) von etwa 30 mg/l bis etwa 150 mg/l Calcium; (b) von etwa 10 mg/l bis etwa 50 mg/l Magnesium; (c) von etwa 150 mg/l bis etwa 700 mg/l Bicarbonate; und (d) von etwa 0,1 mg/l bis etwa 5 mg/l Kalium.

7. Zusammensetzung nach Anspruch 6, worin das besagte Mineralwasser weiterhin umfaßt (e) von etwa 1 bis 20 mg/l Sulfate; (f) von etwa 1 bis 10 mg/l Natrium; (g) von etwa 1 mg/l bis etwa 10 mg/l Chlorid; und (h) von etwa 1 mg/l bis etwa 10 mg/l Nitrate.

8. Zusammensetzung nach einem der Ansprüche 5-7, worin das besagte Mineralwasser Evian® Mineralwasser ist.

9. Verwendung eines Mineralwassers wie in einem der Ansprüche 1 bis 4 angegeben zur Herstellung eines Medikaments zur therapeutischen Behandlung der Haut durch Förderung der Aufnahme eines Nährstoffs und/oder von Sauerstoff in die Haut.

## Revendications

1. Utilisation cosmétique d'une eau minérale ayant une minéralisation d'au moins environ 200 mg/litre, dans laquelle ladite eau minérale comprend au moins 10 mg/litre de calcium et au moins environ 5 mg/litre de magnésium pour promouvoir l'absorption d'un nutriment et/ou d'oxygène dans la peau.

2. Utilisation cosmétique selon la revendication 1, dans laquelle ladite eau minérale comprend (a) d'environ 30 mg/litre à environ 150 mg/litre de calcium ; (b) d'environ 10 mg/litre à environ 50 mg/litre de magnésium ; (c) d'environ 150 mg/litre à environ 700 mg/litre de bicarbonates ; et (d) d'environ 0,1 mg/litre à environ 5 mg/litre de potassium.

3. Utilisation cosmétique selon la revendication 2, dans laquelle ladite eau minérale comprend en outre (e) d'environ 1 à environ 20 mg/litre de sulfates ; (f) d'environ 1 à environ 10 mg/litre de sodium ; (g) d'environ 1 mg/litre à environ 10 mg/litre de chlorures ; et (h) d'environ 1 mg/litre à environ 10 mg/litre de nitrates.

4. Utilisation cosmétique selon l'une quelconque des revendications précédentes, dans laquelle ladite eau minérale est de l'Eau Minérale d'Evian®.

5. Composition comprenant (i) de l'eau minérale ayant une minéralisation d'au moins environ 200 mg/litre, dans laquelle ladite eau minérale comprend au moins environ 10 mg/litre de calcium et au moins environ 5 mg/litre de magnésium ; et (iii) un composé sélectionné à partir de la créatine, de la carnitine, de l'acide pyruvique et d'un sel ou d'esters de ceux-ci acceptables d'un point de vue cosmétique, destinée à être utilisée en tant qu'un produit cosmétique pour promouvoir l'absorption d'un nutriment et/ou d'oxygène dans la peau.

6. Composition selon la revendication 5, dans laquelle ladite eau minérale comprend (a) d'environ 30 mg/litre à environ 150 mg/litre de calcium ; (b) d'environ 10 mg/litre à environ 50 mg/litre de magnésium ; (c) d'environ 150 mg/litre à environ 700 mg/litre de bicarbonates ; et (d) d'environ 0,1 mg/litre à environ 5 mg/litre de potassium.

7. Composition selon la revendication 6, dans laquelle ladite eau minérale comprend en outre (e) d'environ 1 à environ 20 mg/litre de sulfates ; (f) d'environ 1 à environ 10 mg/litre de sodium ; (g) d'environ 1 mg/litre à environ 10 mg/litre de chlorures ; et (h) d'environ 1 mg/litre à environ 10 mg/litre de nitrates.

8. Composition selon l'une des revendications 5 à 7, dans laquelle ladite eau minérale est de l'Eau Minérale d'Evian®.

9. Utilisation d'une eau minérale selon l'une quelconque des revendications 1 à 4 pour la fabrication d'un médicament pour le traitement thérapeutique de la peau par la promotion de l'absorption d'un nutriment et/ou d'oxygène dans la peau.
